# EUROPÄISCHE PATENTSCHRIFT

(11) **EP 0 504 693 B1**
(45) Veröffentlichungstag und Bekanntmachung des Hinweises auf die Patenterteilung: **05.10.1994**
(21) Anmeldenummer: 92103959.0
(22) Anmeldetag: 09.03.1992
(51) Int. Cl.: C07C 253/30, C07C 227/10

(54) **Verfahren zur Herstellung von Aminomethylenverbindungen**
Process for the preparation of aminomethylene compounds
Procédé pour la préparation de composés aminométhyléniques

(30) Priorität: 22.03.1991 DE 4109354
(43) Veröffentlichungstag der Anmeldung: 23.09.1992
(73) Patentinhaber: BAYER AG, 51368 Leverkusen (DE)
(72) Erfinder: Blank, Heinz Ulrich, Dr., W-5068 Odenthal (DE); Kraus, Helmut, Dr., W-5000 Köln 80 (DE)

(56) Entgegenhaltungen:
- EP-A- 0 411 417
- DD-A- 257 067

## Beschreibung

Die Erfindung betrifft die Herstellung von Aminomethylenverbindungen durch Aminomethylenierung von C-H-aciden Verbindungen in einer einstufigen Reaktion.

Aminomethylenverbindungen, wie aminomethylenierte Dinitrile, Cyanessigester und Malonester, sind wichtige C₃- bzw. C₄-Bausteine bei der Synthese von Heterocyclen, wie beispielsweise Pyrazol-, Pyridin- und Chinolinderivaten, die als pharmazeutische Wirkstoffe und Pflanzenschutzmittel Verwendung finden (US 4.620.865).

Es sind eine Reihe von Verfahren zur Aminomethylenierung von C-H-aciden Verbindungen bekannt, die in DE-OS 39 25 720 aufgeführt sind. Diese DE-OS selbst beschreibt die Umsetzungen von C-H-aciden Verbindungen mit Alkoxymethylen-iminiumsalzen bzw. mit Formamidiniumsalzen, wobei Alkoholate als erforderliche Basen eingesetzt werden. Alkoholate sind jedoch teuer und wegen ihrer Reaktivität umständlich zu handhaben. Außerdem besteht die Gefahr, daß beim Einsatz von Estern als den C-H-aciden Verbindungen Umesterungen eintreten. In DD 257 067 wird die Aminomethylenierung von Cyanacetat mit dem DMF-Dimethylsulfat-Addukt in Gegenwart von Natriumcarbonat beschrieben, wobei allerdings nur Ausbeuten von 53 - 61 % der theoretischen Ausbeute erzielt werden.

Es wurde nun gefunden, daß beim Einsatz von Formamidiniumsalzen als Aminomethylenierungsreagentien einfache anorganische basische Verbindungen als erforderliche Base eingesetzt werden können.

Die Erfindung betrifft demnach ein Verfahren zur Herstellung von Aminomethylenverbindungen der Formel 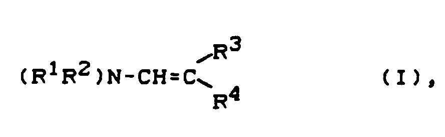
in der
- R¹ und R²: unabhängig voneinander geradkettiges oder verzweigtes C₁-C₈-Alkyl, C₂-C₈-Alkenyl, C₂-C₈-Alkoxyalkyl,C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R¹ und R² gemeinsam mit dem N-Atom, das sie substituieren, einen 5- bis 8-gliedrigen gesättigten oder ungesättigten N-heterocyclischen Ring bilden können, dar ein weiteres Heteroatom aus der Gruppe N, O und S enthalten kann, und
- R³ und R⁴: unabhängig voneinander C₆-C₁₂-Aryl, -NO₂, -CN, -NC, COR⁵, CSR⁵, CO-OR⁵, CO-SR⁵ oder CO-N(R⁵,R⁶) bedeuten, worin R⁵ und R⁶ den für R¹ und R² genannten Bedeutungsumfang annehmen, jedoch unabhängig von R¹ und R² sind und zusätzlich Wasserstoff bedeuten können,
das dadurch gekennzeichnet ist, daß man C-H-acide Verbindungen der Formel 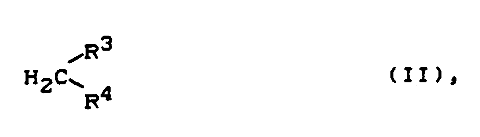
in der
- R³ und R⁴: die genannte Bedeutung haben,
mit Salzen der Formel 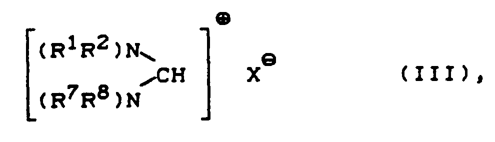
in der
- R¹ und R²: die genannte Bedeutung haben,
- R⁷ und R⁸: unabhängig voneinander und unabhängig von R¹ und R² den für R¹ und R² genannten Bedeutungsumfang haben und
- X^{⊖}: das C₁-C₈-Alkylsulfatanion, das C₆-C₁₂-Arylsulfonatanion, das Tetrafluoroboranatanion, das C₆-C₁₂-Arylsulfatanion, das Chloridanion, das Bromidanion, das Jodidanion, das Hexafluorophosphatanion, das C₁-C₈-Alkylsulfonatanion, das C₁-C₈-Halogenalkylsulfonatanion, das Perchloratanion oder das Hexachloroantimonatanion bedeutet,
in Gegenwart eines (Erd)Alkalihydroxids, -hydrogencarbonats oder -carbonats in einer einstufigen Reaktion bei einer Temperatur von 10 bis 70°C, bevorzugt 20 bis 60°C, umsetzt.

Geradkettiges oder verzweigtes C₁-C₈-Alkyl ist beispielsweise Methyl, Ethyl, Propyl, Isopropyl, Butyl, Isobutyl, tert.-Butyl, die isomeren Amyle, Hexyle, Octyle, bevorzugt die genannten C₁-C₄-Alkylreste.

C₂-C₈-Alkenyl ist Vinyl, Propenyl, Allyl, die isomeren Butenyle, Amylenyle, Hexenyle oder Octenyle, bevorzugt die genannten C₃-C₄-Alkenylreste.

C₂-C₈-Alkoxyalkyl ist beispielsweise Methoxymethyl, Ethoxymethyl, Methoxyethyl sowie weitere Reste aus der Gruppe C₃-C₈-Alkyl, in welchem eine CH₂-Gruppe durch ein O-Atom ersetzt ist.

C₃-C₈-Alkoxyalkenyl ist beispielsweise Methoxyvinyl, Ethoxyvinyl, Methoxyallyl, 2-Methoxy-propenyl und andere aus der Gruppe von C₄-C₈-Alkenyl, worin eine CH₂-Gruppe durch ein O-Atom ersetzt ist.

C₃-C₈-Cycloalkyl ist beispielsweise Cyclopropyl, Methylcyclopropyl, Dimethyl-cyclopropyl, Cyclobutyl, Methylcyclobutyl, Cyclopentyl, Methyl-cyclopentyl, Cyclohexyl, Methyl-cyclohexyl, Dimethyl-cyclohexyl, Cycloheptyl, Cyclooctyl, bevorzugt Cyclopropyl, Cyclopentyl und Cyclohexyl sowie deren Methyl- oder Dimethyl-Derivate.

C₆-C₁₂-Aryl ist beispielsweise Phenyl, Naphthyl oder Biphenylyl, bevorzugt Phenyl.

C₇-C₁₀-Aralkyl ist beispielsweise Benzyl, 1-Phenylethyl, 2-Phenyl-ethyl und weitere dem Fachmann bekannte Reste dieser Art, bevorzugt Benzyl.

Als 5- bis 8-gliedriger gesättigter oder ungesättigter heterocyclischer Ring, dessen Heteroatome 1 oder 2 aus der Gruppe von N, O und S sind, seien genannt; Pyrrol, Furan, Thiophen, Pyrrolidin, Pyrazol, Imidazol, Thiazol, Oxazol, Pyridin, Pyrimidin, Piperazin, Morpholin, Pyran, Azepin, Azocin, Isoxazol, Isothiazol, Pyridazin und Pyrazin.

Es ist dem Fachmann bekannt, daß ungesättigte heterocyclische Ringe einen mehr oder weniger stark ausgeprägten aromatischen Charakter haben können.

Weiterhin können R¹ und R² gemeinsam mit dem N-Atom, das sie substituieren, einen 5- bis 8-gliedrigen gesättigten oder ungesättigten (gegebenenfalls aromatischen) N-heterocyclischen Ring bilden, der ein weiteres Heteroatom aus der Gruppe N, O und S enthalten kann. Solche Systeme sind beispielsweise Pyrrol, Pyrrolidin, Pyrrolin, Pyrazol, Pyrazolidin, Imidazol, Imidazolidin, Thiazol, Thiazolidin, Piperazin, Piperidin, Morpholin, Azepin, Dihydroazocin.

Das C₁-C₈-Alkylsulfatanion ist beispielsweise das Anion von Methylschwefelsäure, Ethylschwefelsäure, Propylschwefelsäure, Isopropylschwefelsäure, Butylschwefelsäure, Isobutylschwefelsäure, eine der isomeren Hexylschwefelsäuren oder Octylschwefelsäuren.

Das C₁-C₈-Alkylsulfonatanion bzw. das C₁-C₈-Halogenalkylsulfonatanion ist beispielsweise das Anion der Methylsulfonsäure, der Trichlormethylsulfonsäure, der Trifluormethylsulfonsäure oder einer Sulfonsäure mit höherem (Halogen)Alkylrest.

Das C₆-C₁₂-Arylsulfonatanion ist beispielsweise das Anion von Benzolsulfonsäure, Naphthalin-sulfonsäure, Biphenyl-sulfonsäure, bevorzugt von Benzolsulfonsäure.

Das C₆-C₁₂-Arylsulfatanion ist beispielsweise das Anion der Phenylschwefelsäure, der Naphthylschwefelsäure oder der Biphenyl-schwefelsäure.

X^{⊖} ist bevorzugt Halogenid oder Alkylsulfat, besonders bevorzugt Chlorid und Methylsulfat.

(Erd)Alkalihydroxide, -hydrogencarbonate und -carbonate sind beispielsweise die von Lithium, Natrium, Kalium, Rubidium, Cäsium, Magnesium, Calcium, Strontium, Barium, bevorzugt die eines Alkalimetalls, besonders bevorzugt die von Natrium oder Kalium.

Die erfindungsgemäße Reaktion kann beispielhaft unter Benutzung von NaOH als Base wie folgt dargestellt werden: 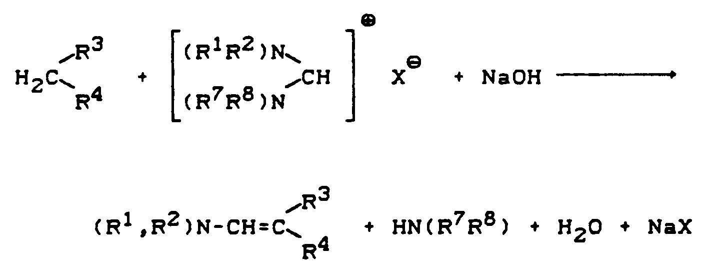
R⁷ und R⁸ haben den Bedeutungsumfang von R¹ bzw. R², sind aber unabhängig von R¹ und R². Sofern R⁷ und R⁸ verschieden von R¹ bzw. R² sind, wählt man solche Reste R⁷ und R⁸, die das sie tragende N-Atom befähigen, als HN(R⁷R⁸) abzuspalten. Dies kann durch einfache Vorversuche festgestellt werden. In bevorzugter Weise gleichen R⁷ und R⁸ jedoch R¹ und R², so daß ein symmetrisches Formamidiniumsalz vorliegt.

In bevorzugter Weise wird eine C-H-acide Verbindung der Formel 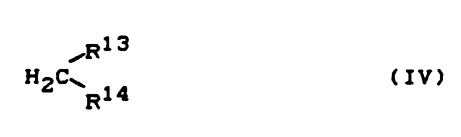
eingesetzt, in der
- R¹³ und R¹⁴: unabhängig voneinander Phenyl, NO₂, CN, COR¹⁵, COOR¹⁵ oder CO-N(R¹⁵,R¹⁶) bedeuten, wobei R¹⁵ und R¹⁶ unabhängig voneinander für Wasserstoff, geradkettiges oder verzweigtes C₁-C₈-Alkyl, Cyclopropyl, Cyclopentyl, Cyclohexyl, Phenyl oder Benzyl stehen und wobei R¹⁵ und R¹⁶ gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann.

In besonders bevorzugter Weise wird eine C-H-acide Verbindung der Formel 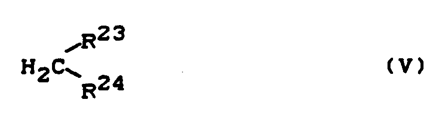
eingesetzt, in der
- R²³ und R²⁴: unabhängig voneinander Phenyl, NO₂, CN, COR²⁵, COOR²⁵ oder CO-N(R²⁵,R²⁶) bedeuten, wobei R²⁵ und R²⁶ unabhängig voneinander Wasserstoff oder geradkettiges oder verzweigtes C₁-C₄-Alkyl bedeuten und wobei weiterhin R²⁵ und R²⁶ gemeinsam mit dem N-Atom, das sie substituieren, Morpholino, Pyrrolidino oder Piperidino bedeuten können.

In weiterhin bevorzugter Weise werden die C-H-aciden Verbindungen mit einem Salz der Formel 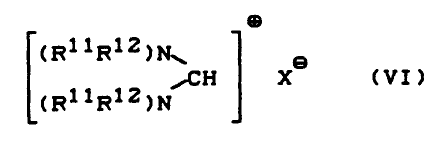
umgesetzt, in der
- R¹¹ und R¹²: unabhängig voneinander geradkettiges oder verzweigtes C₁-C₈-Alkyl, Cyclopropyl, Cyclopentyl, Cyclohexyl, Phenyl oder Benzyl bedeuten und R¹¹ und R¹² weiterhin gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann, und
- X^{⊖}: den oben genannten Bedeutungsumfang annimmt.

Weiterhin werden die C-H-aciden Verbindungen in besonders bevorzugter Weise mit einem Salz der Formel 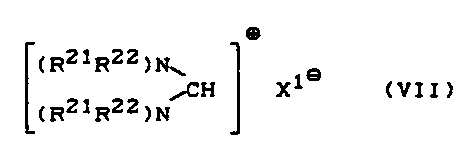
umgesetzt, in der
R²¹ und R²² unabhängig voneinander geradkettiges oder verzweigtes C₁-C₄-Alkyl bedeuten und R²¹ und R²² weiterhin gemeinsam mit dem N-Atom, das sie substituieren, Morpholino, Pyrrolidino oder Piperidino bedeuten können, und 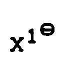
das C₁-C₈-Alkylsulfatanion, das Chloridanion, das Bromidanion oder das Jodidanion bedeutet.

In ganz bevorzugter Weise bedeuten die Substituenten R²¹ und R²² den Methylrest. In weiterhin ganz besonders bevorzugter Weise 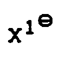
das C₁-C₄-Alkylsulfatanion, das Chloridanion oder das Bromidanion, bevorzugt das Chloridanion oder das Methylsulfatanion.

In bevorzugter Weise wird durch die Umsetzung in Gegenwart von Natrium- oder Kalium-hydroxid, -hydrogencarbonat oder -carbonat als Base durchgeführt.

Die C-H-acide Verbindung, das Salz und die Base werden im allgemeinen im molaren Verhältnis 1:1:1 bis 1:2,5:2 eingesetzt. In bevorzugter Weise wird das Verhältnis 1:1,1:1,05 bis 1:1,7:1,2 gewählt.

Anstelle des reinen Formamidiniumsalzes läßt sich auch das Rohprodukt aus der Umsetzung eines Alkoxymethyleniminiumsalzes mit einem sekundären Amin, das 1 Äquivalent Alkanol, überschüssiges Amin, Dialkylamin-hydroalkylsulfat und Dialkylformamid enthält, verwenden. In einer besonderen Ausführungsform wird die Umsetzung des Alkoxymethylen-iminiumsalzes mit dem sekundären Amin und die Aminomethylenierung in einem Schritt durchgeführt.

Das erfindungsgemäße Verfahren kann grundsätzlich ohne Lösungs- oder Verdünnungsmittel durchgeführt werden, da in der Regel unter den Startmaterialien oder Reaktionsprodukten flüssige Stoffe vorhanden sind, die eine ausreichende Durchmischung des Reaktionsansatzes durch einen Rührer oder Kneter erlauben. Dies gilt insbesondere, wenn das Rohprodukt aus der genannten Umsetzung eines Alkoxymethylen-iminiumsalzes mit einem sekundären Amin eingesetzt wird, das flüssige Stoffe enthält.

Wenn das eingesetzte Salz und die C-H-acide Verbindung nicht mischbar sind, kann man jedoch bevorzugt ein Lösungsmittel verwenden und die daraus resultierende Lösung oder eine durch Rühren gleichmäßig gehaltene Emulsion einsetzen. Es ist weiterhin möglich, beide Komponenten (Salz und C-H-acide Verbindung) simultan zu vorgelegter Base zuzudosieren oder Salz, C-H-acide Verbindung und Base simultan in das Reaktionsgefäß einzudosieren. Bei der Zugabe von Salz und C-H-acider Verbindung oder einem Gemisch beider zu vorgelegter, bevorzugt suspendierter Base werden Nebenreaktionen unterdrückt.

Als Lösungsmittel kommen Kohlenwasserstoffe, wie Toluol, Xylol, Cyclohexan oder Petrolether, halogenierte Kohlenwasserstoffe, wie Methylenchlorid, Alkohole, Carbonylverbindungen oder Ether, aber auch überschüssiges sekundäres Amin aus der Vorstufe zum Einsatz. Solche Lösungsmittel können auch als Gemisch eingesetzt werden.

Die Menge des Lösungs- bzw. Verdünnungsmittels ist für die Reaktion unkritisch und beträgt beispielsweise 30-1000 ml, bevorzugt 50-800 ml, besonders bevorzugt 80-500 ml pro 100 g der aus Salz, C-H-acider Verbindung und Base bestehenden Reaktionspartner.

Es ist dem Fachmann bekannt, daß bei solchen Fest-Flüssig-Reaktionen Phasentransfer-Katalysatoren (PTC) einen reaktionsbeschleunigenden Einfluß haben können. Das erfindungsgemäße Verfahren kann auf den Einsatz von PTC verzichten.

Die Base wird während der einsetzenden Reaktionen verbraucht. Das hierbei entstehende Salz fällt aus und kann von dem im Reaktionsmedium gelösten Produkt auf einfache Weise abgetrennt werden.

Enthält die C-H-acide Verbindung beispielsweise eine Estergruppe oder einen Nitrilsubstituenten, werden dieser unter den milden Reaktionsbedingungen (beispielsweise 0,5 bis 4 h und 20 bis 60°C) nicht angegriffen. Selbst höhere Temperaturen sind kurzzeitig, beispielsweise zum Andestillieren, möglich.

Die Selektivitäten im erfindungsgemäßen Verfahren sind sehr hoch. Die Umsätze sind von der C-H-aciden Verbindung abhängig und können beispielsweise beim Cyanessigester 100 % bei einer Ausbeute von 98 % der theoretischen Ausbeute erreichen. Dadurch ist das Reaktionsprodukt sehr rein und kann für weitere Umsetzungen direkt eingesetzt werden.

### Beispiel 1

Zu einer Suspension von 4,0 g NaOH-Pulver in 140 ml Toluol tropfte man innerhalb 4 min. ein Gemisch aus 25,1 g 97 %igem Tetramethylformamidinium-methylsulfat und 11,4 g Cyanessigsäureethylester. Es wurde noch 4 h bei Raumtemperatur und 1 h bei 50°C nachgerührt und anschließend bei Raumtemperatur abgesaugt. Nach zweimaligem Nachwaschen mit je 10 ml Toluol engte man das Filtrat ein und erhielt 16,8 g Produkt. Nach gaschromatographischer Analyse mit internem Standard wurde ein Gehalt von 98,1 % Dimethylaminomethylencyan-essigsäureethylester festgestellt, entsprechend 98,1 % der theoretischen Ausbeute.

### Beispiel 2

Es wurde wie in Beispiel 1 gearbeitet, jedoch wurden anfangs 0,32 g Tetrabutylammoniumbromid zugesetzt. Man erhielt 94,5 % der theoretischen Ausbeute an Produkt.

### Beispiel 3

Analog Beispiel 1 wurden 3,3 g 85 %iges NaOH-Pulver in 70 ml Toluol mit einer Mischung aus 14,1 g 86,4 %igem Formamidiniumsalz und 5,7 g Cyanessigester umgesetzt. Es wurden 89,3 % der theoretischen Ausbeute an Produkt erhalten.

### Beispiel 4

Man legte 13,8 g Kaliumcarbonat in 100 ml getrocknetem Methanol vor und tropfte ein Gemisch 25,4 g 86,4 %igem Formamidiniumsalz und 9,9 g Cyanessigsäuremethylester zu. Nach 1 h bei Raumtemperatur und 3 h bei 65°C wurde vom kristallinen Salz abgesaugt und eingeengt. Nach der gaschromatographischen Analyse mit internem Standard waren 80,2 % der theoretischen Ausbeute an Produkt neben 9,8 % Cyanessigester vorhanden.

### Beispiel 5

Analog Beispiel 1 wurden 3,3 g 85 %iges NaOH-Pulver in 70 ml Methylenchlorid mit einer Mischung aus 12,3 g 86,4 %igem Formamidiniumsalz und 5,7 g Cyanessigsäureethylester umgesetzt. Man erhielt 85,0 % der theoretischen Ausbeute an Produkt neben 6,2 % nicht umgesetztem Cyanessigester.

### Beispiel 6

Auf 20,9 g Methoxymethyleniminium-methylsulfat wurden bei 0°C 5,5 g Dimethylamin kondensiert. Nach 1 Stunde Rühren bei 20°C zog man im Vakuum überschüssiges Dimethylamin und 70 % des entstandenen Methanols ab. Analog Beispiel 1 wurde das erhaltene Salz mit 11,4 g Cyanesslgsäureethylester und 4 g NaOH in 140 ml Toluol umgesetzt. Man erhielt 93,0 % an Dimethylaminomethylencyanessigsäureethylester neben 1,2 % des entsprechenden Methylesters.

### Beispiel 7

In das vorgelegte Gemisch aus 13,2 g Malonsäuredimethylester, 23,3 g 97 %igem Formamidiniumsalz und 50 ml Methanol wurden bei Raumtemperatur 10,6 g Natriumcarbonat eingetragen und anschließend 4 h unter Rückfluß erhitzt. Nach Versetzen mit 50 ml Toluol wurde das Methanol abdestilliert und bei Raumtemperatur vom Salz abgesaugt. Das eingeengte Filtrat enthielt 40,4 % der theoretischen Ausbeute an Dimethylaminomethylen-malonsäuredimethylester neben 55,2 % nicht umgesetzten Malonesters.

### Beispiel 8

Analog Beispiel 1 wurden 25,1 g 97,0 %iges Formamidiniumsalz mit 13,0 g Acetessigsäureethylester und 6,6 g 85 %igem KOH-Pulver in 140 ml Toluol umgesetzt. Bei 50°C wurde vom Salz abgesaugt und das Filtrat eingeengt. Man erhielt 66,9 % der theoretischen Ausbeute an N,N-Dimethylaminomethylenacetessigsäureethylester neben 30,9 % an Acetessigester.

### Beispiel 9

Analog Beispiel 1 wurden 27,6 g 95,5 %iges Dipyrrolidino-formamidinium-methylsulfat mit 11,4 g Cyanessigsäureethylester und 4,0 g NaOH-Pulver in 140 ml Toluol umgesetzt. Nach der Aufarbeitung wurden 95,5 % Pyrrolidino-methylencyanessigsäure-ethylester erhalten.

### Beispiel 10

In 140 ml Toluol wurden 4,0 g NaOH und 8 g Pyrrolidin vorgelegt. Bei 40°C tropfte man ein Gemisch aus 20,9 g Methoxymethyleniminium-methylsulfat und 11,4 g Cyanessigsäureethylester zu und rührte noch 2 h bei dieser Temperatur. In 93,4 % der theroretischen Ausbeute wurde ein Gemisch aus Pyrrolidino- und Dimethylaminomethylencyanessign (Py : NMe₂ = 10,7 : 1; Et : Me = 10,7 : 1) erhalten.

### Beispiel 11

Zu einer Suspension von 4,4 g NaOH in 140 ml Toluol tropfte man zunächst 11,4 g Cyanessigsäureethylester und anschließend eine Lösung von 14,6 g 98 %igem Tetramethylformamidinium-chlorid in 25 ml DMF. Nach 4 h Rühren bei Raumtemperatur und 1 h bei 50°C saugte man vom Salz ab und engte das Filtrat ein. Es wurden 92,7 % der theoretischen Ausbeute an Dimethylaminomethylencyanessigester erhalten.

## Patentansprüche

1. Verfahren zur Herstellung von Aminomethylenverbindungen der Formel 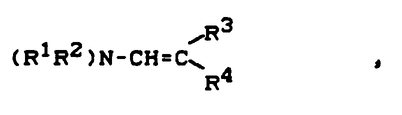 in der
R¹ und R² unabhängig voneinander geradkettiges oder verzweigte: C₁-C₈-Alkyl, C₂-C₈-Alkenyl, C₂-C₈-Alkoxyalkyl,C₃-C₈-Alkoxyalkenyl, C₃-C₈-Cycloalkyl, C₆-C₁₂-Aryl, C₇-C₁₀-Aralkyl oder einen 5- bis 8-gliedrigen gesättigten oder ungesättigten heterocyclischen Ring, dessen Heteroatome 1 oder 2 aus der Gruppe von N, O und S sind, darstellen, wobei weiterhin R¹ und R² gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann, und
R³ und R⁴ unabhängig voneinander C₆-C₁₂-Aryl, -NO₂, -CN, -NC, COR⁵, CSR⁵, CO-OR⁵, CO-SR⁵ oder CO-N(R⁵,R⁶) bedeuten, worin R⁵ und R⁶ den für R¹ und R² genannten Bedeutungsumfang annehmen, jedoch unabhängig von R¹ und R² sind und zusätzlich Wasserstoff bedeuten können,
dadurch gekennzeichnet, daß man C-H-acide Verbindungen der Formel 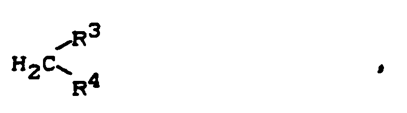 in der
R³ und R⁴ die genannte Bedeutung haben,
mit Salzen der Formel 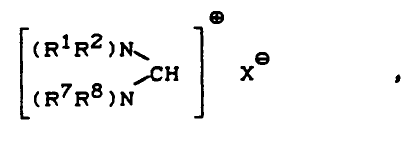 in der
R¹ und R² die genannte Bedeutung haben,
R⁷ und R⁸ unabhängig voneinander und unabhängig von R¹ und R² den für R¹ und R² genannten Bedeutungsumfang haben und
X^{⊖} das C₁-C₈-Alkylsulfatanion, das C₆-C₁₂-Arylsulfonatanion, das Tetrafluoroboranatanion, das C₆-C₁₂-Arylsulfatanion, das Chloridanion, das Bromidanion, das Jodidanion, das Hexafluorophosphatanion, das C₁-C₈-Alkylsulfonatanion, das C₁-C₈-Halogenalkylsulfonatanion, das Perchloratanion oder das Hexachloroantimonatanion bedeutet,
in Gegenwart eines (Erd)Alkalihydroxids, -hydrogencarbonats oder -carbonats in einer einstufigen Reaktion bei einer Temperatur von 10 bis 70°C, bevorzugt 20 bis 60°C, umsetzt.

2. Verfahren nach Anspruch 1, dadurch gekennzeichnet, daß eine C-H-acide Verbindung der Formel 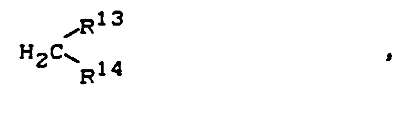 eingesetzt wird, in der
R¹³ und R¹⁴ unabhängig voneinander Phenyl, NO₂, CN, COR¹⁵, COOR¹⁵ oder CO-N(R¹⁵,R¹⁶) bedeuten, wobei R¹⁵ und R¹⁶ unabhängig voneinander für Wasserstoff, geradkettiges oder verzweigtes C₁-C₈-Alkyl, Cyclopropyl, Cyclopentyl, Cyclohexyl, Phenyl oder Benzyl stehen und wobei R¹⁵ und R¹⁶ gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann.

3. Verfahren nach Anspruch 2, dadurch gekennzeichnet, daß eine C-H-acide Verbindung der Formel 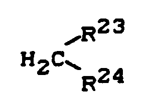 eingesetzt wird, in der
R²³ und R²⁴ unabhängig voneinander Phenyl, NO₂, CN, COR²⁵, COOR²⁵ oder CO-N(R²⁵,R²⁶) bedeuten, wobei R²⁵ und R²⁶ unabhängig voneinander Wasserstoff oder geradkettiges oder verzweigtes C₁-C₄-Alkyl bedeuten und wobei weiterhin R²⁵ und R²⁶ gemeinsam mit dem N-Atom, das sie substituieren, Morpholino, Pyrrolidino oder Piperidino bedeuten können.

4. Verfahren nach Anspruch 1, dadurch gekennzeichnet, daß die C-H-aciden Verbindungen mit Salzen der Formel 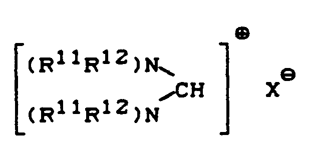 umgesetzt werden, in der
R¹¹ und R¹² unabhängig voneinander geradkettiges oder verzweigtes C₁-C₈-Alkyl, Cyclopropyl, Cyclopentyl, Cyclohexyl, Phenyl oder Benzyl bedeuten und R¹¹ und R¹² weiterhin gemeinsam mit dem N-Atom, das sie substituieren, einen 5- bis 8-gliedrigen gesättigten oder ungesättigten N-heterocyclischen Ring bilden können, der ein weiteres Heteroatom aus der Gruppe N, O und S enthalten kann, und
X^{⊖} das C₁-C₈-Alkylsulfatanion, das C₆-C₁₂-Arylsulfonatanion, das Tetrafluoroboranatanion, das C₆-C₁₂-Arylsulfatanion, das Chloridanion, das Bromidanion, das Jodidanion, das Hexafluorophosphatanion, das C₁-C₈-Alkylsulfonatanion, das C₁-C₈-Halogenalkylsulfonatanion, das Perchloratanion oder das Hexachloroantimonatanion bedeutet.

5. Verfahren nach Anspruch 4, dadurch gekennzeichnet, daß die C-H-aciden Verbindungen mit Salzen der Formel 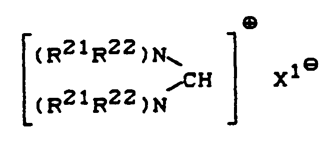 umgesetzt werden, in der
R²¹ und R²² unabhängig voneinander geradkettiges oder verzweigtes C₁-C₄-Alkyl bedeuten und R²¹ und R²² weiterhin gemeinsam mit dem N-Atom, das sie substituieren, Morpholino, Pyrrolidino oder Piperidino bedeuten können, und 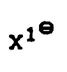 das C₁-C₈-Alkylsulfatanion, das Chloridanion, das Bromidanion oder das Jodidanion bedeutet.

6. Verfahren nach Anspruch 1, dadurch gekennzeichnet, daß in Gegenwart eines Alkalimetallhydroxids, -hydrogencarbonats oder -carbonats, bevorzugt in Gegenwart von Natrium- oder Kalium-hydroxid-, -hydrogencarbonat oder -carbonat gearbeitet wird.

7. Verfahren nach Anspruch 1, dadurch gekennzeichnet, daß die C-H-acide Verbindung, das Salz und die Base in einem molaren Verhältnis von 1:1:1 bis 1:2,5:2, bevorzugt 1:1,1:1,05 bis 1:1,7:1,2, eingesetzt werden.

8. Verfahren nach Anspruch 1, dadurch gekennzeichnet, daß das Formamidiniumsalz in Form seines Rohproduktes eingesetzt wird, wie es aus der Umsetzung eines Alkoxymethylen-iminiumsalzes mit einem sekundären Amin hervorgeht.

9. Verfahren nach Anspruch 8, dadurch gekennzeichnet, daß die Umsetzung eines Alkoxymethylen-iminiumsalzes mit einem sekundären Amin zum Formamidiniumsalz und die Aminomethylenierung einer C-H-aciden Verbindung in einem Schritt durchgeführt werden.

10. Verfahren nach Anspruch 1, dadurch gekennzeichnet, daß man in einem Lösungsmittel arbeitet und das Gemisch aus C-H-aktiver Verbindung und Formamidniumsalz zur Suspension der Base im Lösungsmittel dosiert.

## Claims

1. Process for preparing aminomethylene compounds of the formula 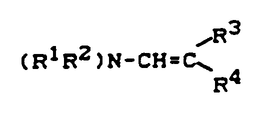 in which
R¹ and R², independently of one another, represent straight-chain or branched C₁-C₈-alkyl, C₂-C₈-alkenyl, C₂-C₈-alkoxyalkyl, C₃-C₈-alkoxyalkenyl, C₃-C₈-cycloalkyl, C₆-C₁₂-aryl, C₇-C₁₀-aralkyl or a 5- to 8-membered saturated or unsaturated heterocyclic ring whose hetero atoms are 1 or 2 from the group consisting of N, O and S, where R¹ and R² together with the N atom which they substitute can furthermore form a 5- to 8-membered saturated or unsaturated N-heterocyclic ring which may contain a further hetero atom from the group consisting of N, O and S, and
R³ and R⁴, independently of one another, denote C₆-C₁₂-aryl, -NO₂, -CN, -NC, COR⁵, CSR⁵, CO-OR⁵, CO-SR⁵ or CO-N(R⁵,R⁶), in which R⁵ and R⁶ adopt the scope of meanings given for R¹ and R² but are independent of R¹ and R² and can additionally denote hydrogen,
characterized in that acidic C-H compounds of the formula 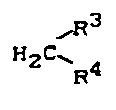 in which
R³ and R⁴ have the meaning given,
are reacted with salts of the formula 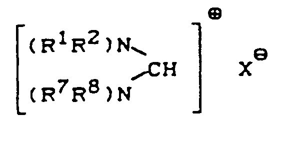 in which
R¹ and R² have the meaning given,
R⁷ and R⁸, independently of one another and independently of R¹ and R² have the scope of meanings given for R¹ and R², and
X^{⊖} denotes a C₁-C₈-alkylsulphate anion, a C₆-C₁₂-arylsulphonate anion, a tetrafluoroboranate anion, a C₆-C₁₂-arylsulphate anion, a chloride anion, a bromide anion, an iodide anion, a hexafluorophosphate anion, a C₁-C₈-alkylsulphonate anion, a C₁-C₈-halogenoalkylsulphonate anion, a perchlorate anion or a hexachloroantimonate anion,
in the presence of an alkali(ne earth) metal hydroxide, alkali(ne earth) metal bicarbonate or alkali(ne earth) metal carbonate in a one-step reaction at a temperature of 10 to 70°C, preferably 20 to 60°C.

2. Process according to Claim 1, characterized in that an acidic C-H compound of the formula 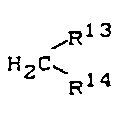 is used, in which
R¹³ and R¹⁴, independently of one another, denote phenyl, NO₂, CN, COR¹⁵, COOR¹⁵ or CO-N(R¹⁵,R¹⁶), where R¹⁵ and R¹⁶, independently of one another, represent hydrogen, straight-chain or branched C₁-C₈-alkyl, cyclopropyl, cyclopentyl, cyclohexyl, phenyl or benzyl and where R¹⁵ and R¹⁶ together with the N atom which they substitute can form a 5- to 8-membered saturated or unsaturated N-heterocyclic ring which may contain a further hetero atom from the group consisting of N, O and S.

3. Process according to Claim 2, characterized in that an acidic C-H compound of the formula 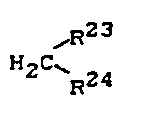 is used, in which
R²³ and R²⁴, independently of one another, denote phenyl, NO₂, CN, COR²⁵, COOR²⁵ or CO-N(R²⁵,R²⁶), where R²⁵ and R²⁶, independently of one another, denote hydrogen or straight-chain or branched C₁-C₄-alkyl and where R²⁵ and R²⁶ together with the N atom which they substitute can furthermore denote morpholino, pyrrolidino or piperidino.

4. Process according to Claim 1, characterized in that the acidic C-H compounds are reacted with salts of the formula 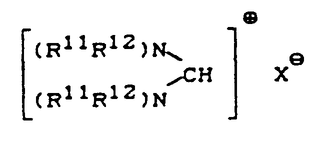 in which,
R¹¹ and R¹², independently of one another, denote straight-chain or branched C₁-C₈-alkyl, cyclopropyl, cyclopentyl, cyclohexyl, phenyl or benzyl and R¹¹ and R¹² together with the N atom which they substitute can furthermore form a 5- to 8-membered saturated or unsaturated N-heterocyclic ring which nay contain a further heteroatom from the group consisting of N, O and S, and
X^{⊖} denotes C₁-C₈-alkylsulphate anion, a C₆-C₁₂-arylsulphonate anion, a tetrafluoroboranate anion, a C₆-C₁₂-arylsulphate anion, a chloride anion, a bromide anion, an iodide anion, a hexafluorophosphate anion, a C₁-C₈-alkylsulphonate anion, a C₁-C₈-halogenoalkylsulphonate anion, a perchlorate anion or a hexachloroantimonate anion.

5. Process according to Claim 4, characterized in that the acidic C-H compounds are reacted with salts of the formula 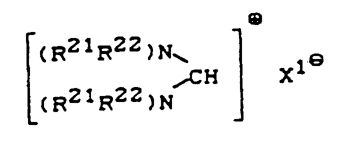 in which
R²¹ and R²², independently of one another, denote straight-chain or branched C₁-C₄-alkyl and R²¹ and R²² together with the N atom which they substitute can furthermore denote morpholino, pyrrolidino or piperidino, and
X^{1⊖} denotes a C₁-C₈-alkylsulphate anion, a chloride anion, a bromide anion or an iodide anion.

6. Process according to Claim 1, characterized in that the reaction is carried out in the presence of an alkali metal hydroxide, alkali metal bicarbonate or alkali metal carbonate, preferably in the presence of sodium hydroxide or potassium hydroxide, sodium bicarbonate or potassium bicarbonate or sodium carbonate or potassium carbonate.

7. Process according to Claim 1, characterized in that the acidic C-H compound, the salt and the base are used in the molar ratio of 1:1:1 to 1:2.5:2, preferably 1:1.1:1.05 to 1:1.7:1.2.

8. Process according to Claim 1, characterized in that the formamidinium salt is used in the form of its crude product such as obtained from the reaction of an alkoxymethylene-iminium salt with a secondary amine.

9. Process according to Claim 8, characterized in that the reaction of an alkoxymethylene-iminium salt with a secondary amine to give the formamidinium salt and aminomethylenation of an acidic C-H compound are carried out in one step.

10. Process according to Claim 1, characterized in that the reaction is carried out in a solvent and the mixture of active C-H compound and formamidinium salt is metered to the suspension of the base in the solvent.

## Revendications

1. Procédé pour la préparation de composés d'aminométhylène répondant à la formule 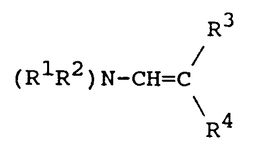 dans laquelle
R¹ et R² représentent, indépendamment l'un de l'autre, un groupe alkyle en C₁-C₈ à chaîne droite ou ramifiée, un groupe alcényle en C₂-C₈, un groupe alcoxyalkyle en C₂-C₈, un groupe alcoxyalcényle en C₃-C₈, un groupe cycloalkyle en C₃-C₈, un groupe aryle en C₆-C₁₂, un groupe aralkyle en C₇-C₁₀, ou encore un noyau hétérocyclique penta- à octogonal saturé ou insaturé, dont les hétéroatomes représentent un ou deux atomes choisis parmi le groupe de N, O et S, dans laquelle, en outre, R¹ et R² peuvent former, conjointement avec l'atome N qu'ils substituent, un noyau N-hétérocyclique penta- à octogonal saturé ou insaturé, qui peut contenir un hétéroatome supplémentaire choisi parmi le groupe N, O et S, et
R³ et R⁴ représentent, indépendamment l'un de l'autre, un groupe aryle en C₆-C₁₂, -NO₂, -CN, -NC, COR⁵, CSR⁵, CO-OR⁵, CO-SR⁵ ou CO-N(R⁵,R⁶) où R⁵ et R⁶ adoptent la portée de signification mentionnée pour R¹ et R², tout en étant indépendants de R¹ et R², et peuvent représenter, en outre, un atome d'hydrogène,
caractérisé en ce qu'on fait réagir des composés C-H-acides répondant à la formule 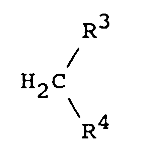 dans laquelle
R³ et R⁴ ont la signification mentionnée,
avec des sels de formule 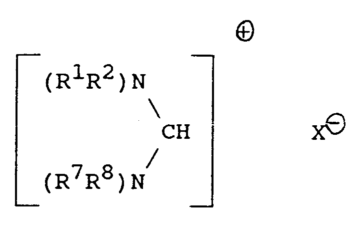 dans laquelle
R¹ et R² ont la signification mentionnée,
R⁷ et R⁸, indépendamment l'un de l'autre et indépendamment de R¹ et R², ont la portée de signification mentionnée pour R¹ et R², et
X⁻ représente l'anion sulfate d'alkyle en C₁-C₈, l'anion sulfonate d'aryle en C₆-C₁₂, l'anion tétrafluoroboranate, l'anion sulfate d'aryle en C₆-C₁₂, l'anion chlorure, l'anion bromure, l'anion iodure, l'anion hexafluorophosphate, l'anion sulfonate d'alkyle en C₁-C₈, l'anion sulfonate d'halogénalkyle en C₁-C₈, l'anion perchlorate ou l'anion hexachloro-antimonate,
en présence d'un hydroxyde, d'un hydrogénocarbonate ou d'un carbonate de métal alcalin ou alcalino-terreux dans une réaction en une seule étape, à une température de 10 à 70°C, de préférence de 20 à 60°C.

2. Procédé selon la revendication 1, caractérisé en ce qu'on met en oeuvre un composé C-H-acide répondant à la formule 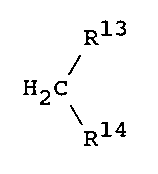 dans laquelle
R¹³ et R¹⁴ représentent, indépendamment l'un de l'autre, un groupe phényle, NO₂, CN, COR¹⁵, COOR¹⁵ ou CO-N(R¹⁵,R¹⁶), où R¹⁵ et R¹⁶ représentent, indépendamment l'un de l'autre, un atome d'hydrogène, un groupe alkyle en C₁-C₈ à chaîne droite ou ramifiée, un groupe cyclopropyle, un groupe cyclopentyle, un groupe cyclohexyle, un groupe phényle ou un groupe benzyle, où R¹⁵ et R¹⁶ peuvent former, conjointement avec l'atome N qu'ils substituent, un noyau N-hétérocyclique penta- à octogonal saturé ou insaturé, qui peut contenir un hétéroatome supplémentaire choisi parmi le groupe N, O et S.

3. Procédé selon les revendication 2, caractérisé en ce qu'on met en oeuvre un composé C-H-acide répondant à la formule 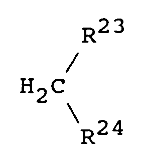 dans laquelle
R²³ et R²⁴ représentent, indépendamment l'un de l'autre, un groupe phényle, NO₂, CN, COR²⁵, COOR²⁵ ou CO-N(R²⁵,R²⁶), où R²⁵ et R²⁶ représentent, indépendamment l'un de l'autre, un atome d'hydrogène ou un groupe alkyle en C₁-C₄ à chaîne droite ou ramifiée, et dans laquelle R²⁵ et R²⁶ peuvent représenter, en outre, conjointement avec l'atome N qu'ils substituent, un groupe morpholino, un groupe pyrrolidino ou un groupe pipéridino.

4. Procédé selon la revendication 1, caractérisé en ce qu'on fait réagir les composés C-H-acides avec des sels de formule 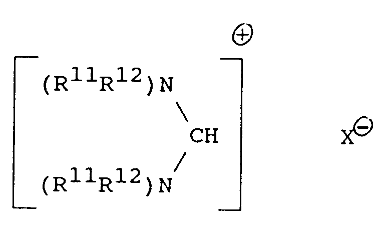 dans laquelle
R¹¹ et R¹² représentent, indépendamment l'un de l'autre, un groupe alkyle en C₁-C₈ à chaîne droite ou ramifiée, un groupe cyclopropyle, un groupe cyclopentyle, un groupe cyclohexyle, un groupe phényle ou un groupe benzyle, et R¹¹ et R¹² peuvent former en outre, conjointement avec l'atome N qu'ils substituent, un noyau N-hétérocyclique penta- à octogonal saturé ou insaturé, qui peut contenir un hétéroatome supplémentaire choisi parmi le groupe N, O et S, et
X⁻ représente l'anion sulfate d'alkyle en C₁-C₈, l'anion sulfonate d'aryle en C₆-C₁₂, l'anion tétrafluoroboranate, l'anion sulfate d'aryle en C₆-C₁₂, l'anion chlorure, l'anion bromure, l'anion iodure, l'anion hexafluorophosphate, l'anion sulfonate d'alkyle en C₁-C₈, l'anion sulfonate d'halogénalkyle en C₁-C₈, l'anion perchlorate ou l'anion hexachloro-antimonate.

5. Procédé selon la revendication 4, caractérisé en ce qu'on fait réagir les composés C-H-acides avec des sels répondant à la formule 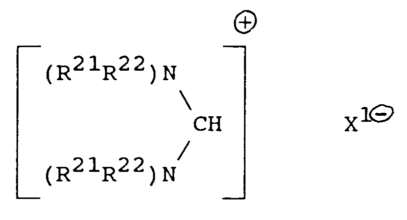 dans laquelle
R²¹ et R²² représentent, indépendamment l'un de l'autre, un groupe alkyle en C₁-C₄ à chaîne droite ou ramifiée, et R²¹ et R²² peuvent représenter, en outre, conjointement avec l'atome N qu'ils substituent, un groupe morpholino, un groupe pyrrolidino ou un groupe pipéridino, et
X^{1⊖} représente l'anion sulfate d'alkyle en C₁-C₈, l'anion chlorure, l'anion bromure ou l'anion iodure.

6. Procédé selon la revendication 1, caractérisé en ce qu'on travaille en présence d'un hydroxyde, d'un hydrogénocarbonate ou d'un carbonate de métal alcalin, de préférence en présence d'hydroxyde, d'hydrogénocarbonate ou de carbonate de sodium ou de potassium.

7. Procédé selon la revendication 1, caractérisé en ce qu'on met en oeuvre le composé C-H-acide, le sel et la base dans un rapport molaire de 1:1:1 à 1:2,5:2, de préférence de 1:1,1:1,05 à 1:1,7:1,2.

8. Procédé selon la revendication 1, caractérisé en ce qu'on met en oeuvre le sel de formamidinium sous forme de son produit brut, tel qu'on l'obtient à partir de la mise en réaction d'un sel d'iminium d'alcoxyméthylène avec une amine secondaire.

9. Procédé selon la revendication 8, caractérisé en ce qu'on effectue la réaction d'un sel d'iminium d'alcoxyméthylène avec une amine secondaire pour obtenir le sel de formamidinium, ainsi que l'aminométhylénation d'un composé C-H-acide en une seule étape.

10. Procédé selon la revendication 1, caractérisé en ce qu'on travaille dans un solvant et on dose le mélange du composé C-H-actif et du sel de formamidinium à des fins de suspension de la base dans le solvant.
